Europäisches Patentamt

European Patent Office

Office européen des brevets

(19)

(11) Publication number : **0 455 744 B1**

(12) **EUROPEAN PATENT SPECIFICATION**

(45) Date of publication of patent specification :
18.08.93 Bulletin 93/33

(51) Int. Cl.⁵ : **C12Q 1/68, C12N 9/12**

(21) Application number : 90903572.7

(22) Date of filing : 31.01.90

(86) International application number :
**PCT/US90/00440**

(87) International publication number :
**WO 90/08839 09.08.90 Gazette 90/19**

(54) THERMOSTABLE POLYMERASE DNA SEQUENCING REACTION CONCENTRATE.

(30) Priority : 06.02.89 US 306423

(43) Date of publication of application :
13.11.91 Bulletin 91/46

(45) Publication of the grant of the patent :
18.08.93 Bulletin 93/33

(84) Designated Contracting States :
DE FR GB

(56) References cited :
EP-A- 0 258 017
WO-A-88/05470
Proc. Natl. Acad. Sci., vol. 85, 1988 (USA),
Michael A. Innis et al "DNA sequencing with
Thermus aquaticus DNA polymerase and di-
rect sequencing of polymerase chain reac-
tion-amplified DNA", pages 9436-9440

(73) Proprietor : EASTMAN KODAK COMPANY
343 State Street
Rochester, New York 14650-2201 (US)

(72) Inventor : VIZARD, Douglas, Lincoln
663 Meriden Road
Cheshire, CT 06410 (US)
Inventor : HADMAN, Martin
(current address unknown)
x (US)

(74) Representative : Nunney, Ronald Frederick
Adolphe et al
Kodak Limited Patent Department Headstone
Drive
Harrow Middlesex HA1 4TY (GB)

## Description

Field of the Invention

This invention relates to DNA sequencing, a reaction concentrate for sequencing, a primer for the sequencing and a kit comprising the reaction concentrate.

## BACKGROUND OF THE INVENTION

There are several method for sequencing DNA. One of the most frequently used is that of Sanger et al reported in Proc. Natl. Acad. Sci. USA volume 74, No. 12, pp. 5463-5467. Sanger's method is based on the inhibitory activity of nucleotide analogues such as dideoxy nucleotide triphosphates (ddNTPs where N is thymine, guanine, cytosine or adenine) on the ability of DNA polymerase to catalyze the growth of a oligonucleotide chain.

For example, when a primer and template are incubated with DNA polymerase in the presence of a mixture of ddTTP and dTTP, as well as the other three deoxyribonucleosides triphosphates (dNTP wherein N is G, A and C) one of which is labeled with a radio-active nucleotide, a mixture of fragments will be synthesized all having the same 5' end with ddT residues at the 3' end is obtained. When this mixture is fractionated by electrophoresis on denaturing acrylamide gels, the pattern of bands show the distribution of dTs in the newly synthesized DNA. By using analogous terminators for other nucleotides in separate incubations and analyzing the samples in parallel on a gel, a pattern of bands is obtained from which the sequence can be deduced.

In general, a DNA fragment to be sequenced is spliced into a particular site in the DNA of a sequencing vector, such as M13 phage, using standard recombinant DNA splicing methods. The resulting recombinant phage DNA is used to infect bacterial cells, and virus particles are produced. These particles contain only one of two strands of DNA.

The single strands of DNA are purified from the viruses. They are mixed with pieces of DNA referred to as primers. Primers are complementary to a region of the M13 DNA near the position where the DNA to be sequenced has been inserted. The primer forms base pairs with the M13 DNA creating a short double-stranded region of DNA that serves as a primer for synthesis. A large number of these partially double-stranded DNA molecules are mixed with DNA polymerase and a radioactive dNTP. DNA polymerase synthesizes radioactive DNA from the M13 template (beginning at the 3' end of the short double-stranded region). The synthesis ultimately involves the DNA region that is to be sequenced and which acts as a template.

The synthesis can be terminated by including a ddNTP in the biochemical reaction. If the ddNTP is ddTTP, the new radioactive chain will stop after it would normally have added a T. Normal dTTP is included in the reaction mix to allow some DNA synthesis before a ddTTP stops the synthesis. Since there are many T's in a nucleotide sequence the stops will not always be in the same place. Thus by carefully adjusting the amount of analog in the reaction it is possible to create a collection of radioactive DNA fragments that begin at the 3' end of the primer and stop at the various positions where T's occur. This procedure is repeated in three other test tubes comprising all of the necessary dNTPs and an analog ddGTP, ddCTP or ddATP. This results in four separate collections of DNA fragments. Every fragment in each tube starts at the same place and ends at different distances in a G, A, T or C dependent on the specific ddNTP included.

The lengths of the molecules are measured by' gel electrophoresis under conditions that are so precise that they can separate DNA sequences differing by only one nucleotide. The four separate collections are electrophoresed next to each other and after electrophoresis a photographic film is exposed to the radioactive emissions of the DNA in the prepared electrophoretic gel. By analysis of the developed film one is able to read the DNA sequence directly from the photographic film.

This method normally involves many different steps that must be carefully carried out in order to achieve precise and accurate sequence analyses. The elimination of any of the steps would improve the method. For example, the method as generally performed requires separate mixing of all the chemical components involved at the time of use. That is the buffer, enzyme, nucleotides, nucleotide analogues and oligonucleotide primer are mixed together separately for each nucleotide G, T, A and C.

## SUMMARY OF THE INVENTION

The present invention provides a nucleotide sequencing reaction concentrate comprising:
a) sufficient thermostable polymerase to provide from 100 to 500 I.U./mL;
b) from 0.3 to 30 mM of a reducing agent selected from dithiothreitol and β-mercaptoethanol;
c) sufficient phosphate buffer to maintain a pH of about 7.5;

d) at least 40% glycerol;

e) from 15 to 150 μM dGTP or daGTP (where "a G" represents 7-deazaguanine);

f) from 15 to 150 μM dATP;

g) from 15 to 150 μM dTTP;

h) from 10 to 18 μM dCTP; and

i) one member selected from the group consisting of:

    i) sufficient ddGTP to provide a ddGTP to dGTP or daGTP ratio of about 5;

    ii) sufficient ddATP to form a ddATP to dATP ratio of about 33;

    iii) ddTTP in a sufficient amount to form a ddTTP to dTTP ratio of about 28; and

    iv) ddCTP in a sufficient amount to form a ddCTP to dCTP ratio of about 22.

Use of the reaction concentrate of this invention eliminates the need to mix all the components needed for sequencing a nucleotide chain at the time of use. The reaction concentrate comprises all of the essential components in a premixed package that can be stored and reused during several cycles. One advantage of the reaction concentrates is the simplicity of the method involving execution of the sequencing reaction steps. Such steps involve the fewest number of steps of all current sequencing procedures and approaches a desirable "single-step" protocol for sequencing.

In a preferred embodiment the reaction concentrate comprises, in addition to the components listed above:

a) from 175 to 700μg/mL of concentrate of a protein selected from the group consisting of gelatin and bovine serum albumin;

b) at least 25 mM of potassium glutamate;

c) from 0.1% to 0.35% V/V of a non-ionic surfactant; and

d) from 9 to 35 mM of a magnesium salt such as magnesium acetate or magnesium chloride.

In this embodiment the reaction concentrate can be stored at temperatures as low as -80°C to -20°C without any deleterious effect on the ability of the components to react in the chemical reactions involved in nucleotide sequencing.

In practice, the dCTP concentration in the reaction can be reduced to 3μM and still support sufficient enzyme reaction kinetics. This is the only one of the four nucleotides that can be reduced to that level. Consequently, dCTP is the recommended target nucleotide used for radioactive labeling of the synthesized strand. Such a low concentration of the target nucleotide is essential for efficient labeling, especially if the $^{35}$S thioester of dCTP is to be used. Other radioactive nucleotides can be used with substantially reduced efficiency.

## DETAILS OF THE INVENTION

The essential components of the reaction concentrates are 100 to 500, preferably 250 I.U./mL of a thermostable polymerase, preferably <u>Thermus</u> aquaticus polymerase (Taq pol); dithiothreitol (DTT); phosphate buffer; magnesium salt; glycerol; the deoxynucleotide triphosphates dNTPs wherein N is guanine (G), 7-deazaguanine (aG), adenine (A), thymine (T) and cytosine (C); and a ddNTP (dideoxy NTP wherein N is as previously defined) to terminate the G, A, T and C reactions.

In one embodiment this invention provides four identical reaction concentrates differing only in the concentration of each dNTP, the specific ddNTP and the ratio of ddNTP to dNTP. The specific ddNTP would depend on which nucleotide is designated as the terminating nucleotide in any polymeric sequence. According to the Sanger method that would be G, A, T or C.

In addition to the foregoing essential elements, reaction concentrate can optimally contain a) a protein; b) a non-ionic detergent; and c) potassium glutamate.

The reaction concentrate can be made even more convenient to the ultimate user by including therein a primer as well as the radioactive labeled dNTP. However, the short half life of radioactive dNTPs makes the inclusion thereof impractical.

Reaction concentrates can be constructed that contain nearly all of the essential components to DNA sequencing. This presents the opportunity for a user to simply add a solution of DNA to be sequenced to a mixture of reaction concentrate, incubate the mixture at elevated temperature, terminate and analyze the reaction products as in contemporary practice. Such method eliminates many steps and is far more amenable to automation than any contemporary sequencing methodology.

The thermostable enzyme is present in the concentrate in sufficient amount to provide an activity of about 0.5 I.U per base-specific reaction. I.U. is the amount of enzyme required to catalyze a polymerization of 10 micromoles of substrate nucleotide per 30 minutes under a broad pH range and a temperature of 70°C for this enzyme.

As used herein, the term "thermostable enzyme" refers to an enzyme which is stable to heat and is heat resistant and catalyzes polymerization of nucleotides to form a nucleotide sequence that is complementary to the unknown nucleotide sequence. Generally, the synthesis will be initiated at the 3' end of each primer and will proceed in the 5' to 3' direction along the template strand thereby proceeding into the unknown nucleotide sequence of the sequencing vector, until synthesis is terminated by a ddNTP, producing oligonucleotides of different lengths. There may be a thermostable enzyme, however, which initiates synthesis at the 5' end and proceeds in the other direction, using the same process as described above.

The preferred thermostable enzyme herein is a DNA polymerase isolated from Thermus aquaticus (Taq pol). Various strains thereof are available from the American Type Culture Collection, Rockville, Maryland, and is described by T.D. Brock, J. Bact. (1969) 98:289-297, and by T. Oshima, Arch. Microbiol. (1978) 117:189-196. One of these preferred strains is strain YT-1.

Our experiments have shown that Taq Pol

1. does not react at low temperatures and can be stored in elevated salt and appropriate reaction buffer conditions at -20°C;

2. is relatively free of contaminating nucleases and phosphatases; and

3. maintains appreciably activity in relatively high concentrations of glycerol. These observations made the present reaction concentrates possible.

The thermostable enzyme may also be produced by recombinant DNA techniques, as the gene encoding this enzyme has been cloned from Thermus aquaticus genomic DNA. The complete coding sequence for the Thermus aquaticus, (Taq) polymerase can be derived from bacteriophage CH35:Taq#4-2 on an approximately 3.5 kilobase (kb) BglII-Asp$^{718}$ partial restriction fragment contained within an ~18 kb genomic DNA insert fragment. This bacteriophage was deposited with the American Type Culture Collection (ATCC) on May 29, 1987 and has accession no. 40,336. Alternatively, the gene can be constructed by ligating an -750 base pair (bp) BglII-HinIII restriction fragment isolated from plasmid pFC83 (ATCC 67,422, deposited May 29, 1987) to an ~2.8 kb HinIII-Asp718 restriction fragment isolated from plasmid pFC85 (ATCC 67,421, deposited May 29, 1987). The pFC83 restriction fragment comprises the amino-terminus of the Taq polymerase gene while the restriction fragment from pFC85 comprises the carboxyl-terminus. Thus, ligation of these two fragments into a correspondingly digested vector with appropriate control sequences will result in the translation of a full-length Taq polymerase.

These parameters were determined experimentally. Less than the 0.5 I.U. per base-specific reaction results in artificial termination of nucleotide chains. Greater than 2 I.U. per base-specific reaction is unnecessary.

The protein can be bovine serum albumin (BSA) or gelatin. Taq pol enzymatic activity is improved by a protein environment. The protein also enhances the shelf life of the reaction concentrate. The presence of the protein also seems to avoid the inclusion of artifacts in a polymerized nucleotide sequence in which Taq pol catalyzed polymerizations. Amounts of 175-700μg/mL of protein in the reaction concentrate, preferably 350μg/mL, are desirable. Concentrations below 175μg/mL result in instability. Concentrations greater than 700μg/mL are unnecessary.

A reducing agent is essential. Dithiothreitol (DTT) is preferred. It maintains the sulfhydryl groups of Taq pol in a reduced state. This reduced state is necessary for Taq pol activity. Other reducing agents include β-mercaptoethanol. Amounts from 0.3 to 10 mM of DTT are useful, preferably 3.5 mM. Concentrations outside of this range result in diminished rates of polymerization. Concentrations above this range are unnecessary.

Commercial preparation of Taq pol usually contains small amounts (about 0.1 mM) of EDTA as an aid for preservative purposes. However, more than 1mM EDTA would be detrimental to the reaction concentrate because EDTA is a metal scavenger and would therefore have an affect on magnesium ion activity which is essential for Taq pol activity.

The reaction concentrate preferably contains up to 17.5 mM of a magnesium salt such as magnesium chloride or magnesium acetate (±50%). Magnesium acetate is preferred. It is essential that it is present at about a 5 mM concentration in the final reaction mix. Amounts in the ranges of ±50% of 5 mM magnesium acetate will be effective. Above or below these ranges the magnesium will have a deleterious affect on the enzyme activity.

Potassium glutamate (K glutamate) also enhances Taq pol enzymatic activity. It is essential for long term storage of the reaction concentrate. Long term storage requires a minimum amount of 25 mM of the potassium glutamate. Higher concentrations lead to an unacceptable salt concentration that would have a deleterious affect on the electrophoretic analysis carried out in the sequencing method provided by this invention.

Potassium hydrogen phosphate and potassium dihydrogen phosphate ($K_2HPO_4$ and $KH_2PO_4$) should be present in an amount sufficient to establish a pH of about 7.5. Phosphate buffer is a thermally stable buffer. It is able to maintain essentially the same pH over a temperature range of -20° to 75°C. It is necessary to maintain the pH constant in storage as well as in use so that Taq pol activity is constant from storage to use.

Glycerol is an antifreeze. Repeated freezing of any enzyme must be prevented to maintain enzyme activity as the reaction concentrate goes through the expected cycles of thawing for use and then restorage for future uses. We have found that at storage temperatures of -70°C to -20°C at least 40% glycerol must be added to the reaction concentrate to prevent repeated freezing. Up to 80% of glycerol could be used without adversely affecting Taq pol activity.

The reaction concentrate can comprise from 0.1 to 0.4% of a non-ionic detergent. Examples include ethoxylated fatty alcohol ethers and lauryl ethers, ethoxylated alkyl phenols, octylphenoxy polyethoxy ethanol compounds, modified oxyethylated and/or oxypropylated straight-chain alcohols, polyethylene glycol monooleate compounds, polysorbate compounds, and phenolic fatty alcohol ethers. More particularly preferred are TRITON X-100, Tween 20, from ICI Americas Inc., Wilmington, DE, which is a polyoxyethylated (20) sorbitan monolaurate, and Iconol™ NP-40, from BASF Wyandotte Corp., Parsippany, NJ, which is an ethoxylated alkyl phenol(nonyl).

Each of the four dNTPs are also essential components of each concentrate. Each of the four concentrates must contain one, and only one, of the ddNTPs according to the termination reaction designed into each reaction concentrate package.

In one embodiment the materials of the present invention can be provided to the ultimate user as a kit in several embodiments. Consistent with the spirit of this invention of user convenience, it is desirable to provide a kit in which the kit components can be used directly without dilution steps. Thus the most convenient kit would be designed to allow the user, based on the practice in many biochemistry labs, to take approximately 2µL of the reaction concentrate for direct use.

In one embodiment the kit can comprise distinct and separately packaged chemicals which may be labeled 1) primer and 2) separate reaction concentrates G, A, T and C.

An optional user variable package can include a sequencing vector or a control DNA and a radioactive labeled dNTP. The control has a known DNA sequence which allows the user to confirm that the kit is operative.

The sequencing vector comprises the nucleotide chain to be sequenced. The most widely used sequencing vectors have been derived from bacteriophage M13. This vector is widely available commerically in various formats usually differing only by their multiple cloning regions. Widely used versions of this vector include M13mp18 and mp19. Other commercially available sequencing vector templates include pUC, pIBI, Bluescript and others. If the primer described hereinafter is used, any sequencing vector having a lacZ nucleotide sequence can be used.

Alternatively, the user may choose to supply the primer and not use the supplied primer. The user may also choose to independently label the primer with a radioactive or fluorescent label. In this situation, additional use of radioactive labels is unnecessary. As long as the primer concentration and binding ability is substantially unmodified, the reaction concentrations of this invention will be useful.

Commercially supplied radioactive nucleotides are sold at a concentration range of about 3 to 16 µM in water. This amount (about 1µL) is added as a user variable where that amount is then further diluted by adding a certain amount of the user variable part of the composition to each of 4 different reaction concentrates. That is, the 1 µL of radioactivity as a user variable is co-mixed with the user's DNA or control DNA, water and 5 µL of primer to total 20 to 23 µL, and is divided 4 times to end up with 2.5 µCi per 5 µL to which 2 µL of reaction concentrate are added, giving four final reaction mixtures of 7 µL each.

The required amount of radionucleotide for each of the 4 reactions is based on completing the sequence analysis in 24 hours with use of a preferred isotope. The preferred isotope is alpha-labeled $^{35}$S dCTP although others such as $^{32}$P could be used. The preferred range would therefore be 1-2µL of alpha-labeled $^{35}$S dCTP in the kit. This provides about 10µCi/µL at a specific activity of 400-600 Ci per mM of $^{35}$S dCTP.

The user can supply the radioactive dNTP or it can be included in each of the reaction concentrates. When the user provides both the sequencing vector and the radioactive dNTP, this constitutes the user variables.

One version of a user variable is presented below. These variables are supplied by the user. Optionally they may be supplied as part of a kit.

```
                                                    Concentration in
                                                    Final Reaction
User Variable Concentrate                              Mixture
1µgM13mp18 or a user selected
  sequencing vector amounting to
  0.4 pmoles of the
DNA to be sequenced................. 0.1 pmoles/7µL
1µL alpha-labeled dCTP.............. 2.5µCi/7µL
Plus water to a final
volume not to exceed 18 µL
```

The primer concentrate package comprises an appropriate concentration of an oligonucleotide primer in a volume of 5µL.

Essential to Taq pol sequencing is the extension of an appropriate primer. Elevated temperature of synthesis does not permit effective use of "universal primer A', which is a 17 base oligomer described herein below. Using longer oligomers does permit "spontaneous" priming of template DNA at elevated temperatures, and using such an appropriate primer obviates the need for the priming reaction essential to other DNA sequencing methodologies. The phrase "spontaneous" means that the priming reaction occurs without intervention by the user.

In a kit according to this invention the primer includes the commonly used universal primer region of M13mp18, but also includes additional bases in the surrounding sequence, taking advantage of more stable G and C residues. This oligomer is compatible with all lacZ based vectors (M13, pUC, pIBI, Bluescript and others). The oligomer sequence is:

$$5' \text{ CCCAGTCCACGACGTT}\underline{\text{GTAAAACGACGGCCAGT}}\text{G } 3'$$

The underlined region is the traditionally used universal primer A region. If primers shorter than 33 mer are used, excessive molar amounts are required for spontaneous priming. Such excessive amounts will result in non-specific priming leading to ambiguous sequencing results.

Longer primers can also be used. However, they seriously compromise sequencing results. Extending in the 5' direction leads to G rich sequence inclusions, resulting in ambiguous electrophoretic mobilities caused by nucleotide structure. Extending in the 3' direction interferes with the cloning sites of the M13 vector. A 3 molar excess of the above primer to target DNA gives full spontaneous priming. Greater than a 10 molar excess produces background on sequencing gels, interpreted as non-specific priming. Limiting the total amount of primer to the specified 0.3 moles per 7µL reaction limits the total number of possible primed target DNA molecules. Hence, the user can use excessive amounts of DNA without depleting the nucleotide pools (up to 0.3 pmoles of primer at 200 number-average bases per extension is 15 pmoles of dCTP consumption for an average unknown DNA sequence, whereas a 7µL reaction containing 3µM dCTP, amounts to 21 pmoles available for consumption).

A preferred primer concentrate contains:

```
                                                    Concentration in
                                                    Final Reaction
                                                       Mixture
1 mM Tris/HCl (pH8)................ 0.18 mM
0.1 mM EDTA.........................0.018 mM
0.24 µM A-33 primer................0.3 pmoles/7µL
Total Volume 5 µL
```

The third separately labeled group of packages are the reaction concentrates. The reaction concentrate may be provided in the kit as four separate labeled packages, i.e. concentrate G, concentrate A, concentrate T and concentrate C. Each of the reaction concentrates will contain the same chemicals at the same concentrations except the concentrations of dNTPs and ddNTPs in each will be different. The ratio of ddNTP to the dNTP have been adjusted experimentally for each concentrate to achieve balanced lane distribution of the radioactive labeled dNTP. Balanced lane distribution leads to uniform appearance of photographs showing the oligonucleotide fragment distribution. This results in more precision and accuracy in reading sequence from the photographic film.

A very useful reaction concentrate is presented below.

```
                                            Concentration of
                                            Final Reaction
                                            ___Mixture____

0.25µ/µL Taq pol.......................... 0.5µ/7µL
350µg/ml BSA.............................. 100 µg/ml
3.5 mM DTT................................ 1 mM
17.5 mM Mg Acetate........................ 5 mM
25 mM K Glutamate......................... 7.1 mM
13.1 mM K₂HPO₄............................ 3.75 mM
4.4 mM KH₂PO₄............................. 1.25 mM
0.35% Triton X-100........................ 0.1%
40% Glycerol.............................. 11.4%
    and a dNTP/ddNTP concentrate selected from Table I
    below.
Total Volume 2 µL
```

## Table I
### dNTP Concentrations (µM) for Reaction Concentrates

|        | G      | A     | T    | C    |
|--------|--------|-------|------|------|
| daGTP  | 22.75  | 70    | 70   | 70   |
| dATP   | 105    | 17.5  | 105  | 105  |
| dTTP   | 52.5   | 52.5  | 17.5 | 52.5 |
| dCTP   | 10.5   | 10.5  | 10.5 | 10.5 |
| ddGTP  | 113.75 | –     | –    | –    |
| ddATP  | –      | 577.5 | –    | –    |
| ddTTP  | –      | –     | 490  | –    |
| ddCTP  | –      | –     | –    | 231  |

The final reaction concentrations dNTP and ddNTP are shown in Table II based on the concentrations of the various chemical components in each package of the above kit. The nucleotides daGTP and dGTP are interchangeable.

## Table II
## Final Reaction Concentrations in µM

|        | G    | A   | T   | C  |
|--------|------|-----|-----|----|
| daGTP  | 6.5  | 20  | 20  | 20 |
| dATP   | 30   | 5   | 30  | 30 |
| dTTP   | 15   | 15  | 5   | 15 |
| dCTP   | 3    | 3   | 3   | 3  |
| ddGTP  | 32.5 | –   | –   | –  |
| ddATP  | –    | 165 | –   | –  |
| ddTTP  | –    | –   | 140 | –  |
| ddCTP  | –    | –   | –   | 66 |
|        |      |     |     |    |
| dd/d ratio | 5 | 33 | 28 | 22 |

In another embodiment of the kit the reaction concentrates G, A, T and C can also include the primer and/or the radioactive labeled dNTP.

In another embodiment of the kit, a single reaction concentrate may include all chemicals except the dNTPs and/or the selected ddNTP. The dNTPs and selected ddNTP could be separately packaged according to Table I. Each of these separately packaged dNTP mixtures would be added to the reaction concentrate, as appropriate, at the time of use.

In manufacturing the reaction concentrate, the order of addition is relatively unimportant, except the enzyme should be added last. Generally the order of addition is: salts and buffers, nucleotides, other reagents, glycerol and enzyme with thorough mixing upon each addition, carried out at ice temperature.

Using the kit described above, in any of the described formats, the method of sequencing would generally comprise the steps of:

a) providing a sequencing vector comprising an unknown nucleotide sequence adjacent to a 3' end of a known nucleotide chain in the vector;

b) mixing the vector with a primer and a radioactive labeled dNTP; wherein the primer has a nucleotide sequence which is complementary to a portion of the known vector DNA sequence that is adjacent to the unknown DNA sequence;

c) dividing the mixture b) into four equal volumes in four different vessels labeled G, A, T and C;

d) providing a reaction mixture by;

    i) adding to the vessel designated G a reaction concentrate according to the present invention comprising ddGTP;

    ii) adding to the vessel designated A a reaction concentrate according to the present invention comprising ddATP;

    iii) adding to the vessel designated T a reaction concentrate according to the present invention comprising ddTTP; and

    iv) adding to the vessel designated C a reaction concentrate according to the present invention comprising ddCTP;

e) incubating the four vessels G, A, T and C at a temperature of 70-74°C from 15 to 30 minutes;

f) terminating the reactions by the addition of 3 to 5 µL of a reaction stop mixture to each of the four tubes;

g) separating electrophoretically the oligo-nucleotides formed in e) according to length; and

h) exposing the electrophoretic separations to a photographic film which is sensitive to the radiation generated by the radioactive dNTP to establish the nucleotide sequence of the unknown nucleotide chain.

In a typical reaction, a user provides a quality single-stranded DNA preparation, typically 1µg (0.4 pmoles) of M13mp18 DNA, at a concentration of >100µg/ml. The user combines the DNA, 5µL of the Primer/Buffer, water and radioactive nucleotide (usually 1µCi of $^{32}$P or 10µCi of $^{35}$S, alpha-labeled dCTP at >600 Ci/mM), to a total volume of 21µL. This DNA mixture is divided equally (5µL) among four tubes (preferably 0.5ml microfuge tubes), designated for the essential G-, A-, T- and C-specific reactions. These tubes are placed at 70-74°C.

Then 2μL of the appropriate specific Reaction Concentrate is mixed into the designated tubes. The tubes are capped and the incubation continued at the 70-74°C for 30 minutes.

The reaction can be terminated with the addition of 3μL of reaction stop mixture. Reaction stop mixtures are well known. EPO 0 258 017 describe such mixtures as including EDTA to chelate magnesium ion, phenol, SDS or CHCl$_3$. A useful mixture in formamide solvent is

0.02 M EDTA;
0.07 M Tris (to neutralize EDTA);
0.05% w/v brophenol blue; and
0.02% w/v xylene cyanol.

The latter two components are anionic chromophores to provide a visual assessment of the progress of electrophoresis.

While the main advantage of using Taq pol for sequencing resides is its ability to synthesize at high temperature (minimizing structural interference by the template strand), a considerable benefit realized by this invention is its adaptability to double-stranded DNA (dsDNA) sequencing including supercoil sequencing.

The principle of double-stranded or supercoiled sequencing depends upon a fraction of supercoils being irreversibly denatured to a single-stranded state, thereby being subject to primed synthesis essential to sequencing reactions.

A significant fraction of irreversibly denatured supercoils is obtained by alkaline denaturation and acid neutralization of supercoiled DNA. Normal double-stranded DNA may be similarly treated, resulting is higher fraction of single-stranded DNA.

In practice, alkaline denaturation and subsequent acid neutralization of supercoil or double-stranded DNA will result in an accumulated salt concentration that is high and a pH that is relatively undefined because of the relatively small, inaccurate volumes necessitated by this process. It is generally necessary to repurify the treated DNA to eliminate the salt and control the pH, since all other extant sequencing protocols will not tolerate elevated salts and/or a buffered pH that would overwhelm the buffer system (usually Tris) utilized in those methods and necessary to the activity of those enzymes.

Phosphate buffer has the highest degree of thermal stability of any commonly used buffer. The phosphate buffer was chosen for the reaction concentrate since the pH must be controlled for the benefit of long-term storage at temperatures as low as -80°C, and reaction temperatures as high as 80°C. Conversely, Tris buffer has among the lowest thermal stability of any buffer, the pK of which lowers more than two full units from 0°C to 70°C. Hence, the supercoiled sequencing reaction can be carried out with the method of this invention without having to repurify the denatured DNA.

This is accomplished by:

a) providing a double-stranded sequencing vector having a lacZ nucleotide sequence into which dsDNA having an unknown nucleotide sequence has been inserted adjacent to a 3' end of a known nucleotide chain in the vector;

b) denaturing the vector with an alkaline solution to form ssDNA;

c) neutralizing the denatured DNA containing solution; and

d) carrying out steps b) through h) of the nucleotide sequencing method described hereinbefore.

In a typical example of the above sequencing method, a several μL volume of supercoiled DNA (in a dilute buffer, e.g. 1mM Tris, 0.1mM EDTA, pH 8) is denatured with 1μL of 0.1M KOH at 37°C for 2 minutes. More severe treatment is usually not necessary. On ice, the primer and the radioactive dNTP are added, and the denatured DNA is neutralized with the addition of 1μL of 0.2M TrisCl. Water is then added to obtain a 21 to 23 μL volume. The final salt and pH will generally be inaccurate. However, the method of this invention can be used without further adjustments. In theory, the resulting salt system consists of a 30-50mM Tris buffer at a pH of 7-9. Upon heating this solution to 70°C for enzymatic reaction, the influence of the Tris buffer becomes negligible, since the phosphate buffer contributed by the reaction concentrate stabilizes the final pH of the entire mixture. Normal single-stranded sequencing occurs unperturbed, and supercoil sequencing occurs at the expected reduced efficiency, commensurate with the fraction of irreversibly denatured supercoils. Hence, supercoil sequencing is accomplished without repurification of the denatured supercoiled DNA, using essentially the same methodology as single strand sequencing.

The invention has been described in detail with particular reference to preferred embodiments thereof, but it will be understood that variations and modifications can be effected within the scope of the invention.

## Claims

1. A nucleotide sequencing reaction concentrate comprising:

a) sufficient thermostable polymerase to provide from 100 to 500 I.U./mL;
b) from 0.3 to 30 mM of a reducing agent selected from dithiothreitol and β-mercaptoethanol;
c) sufficient phosphate buffer to maintain a pH of about 7.5 both in storage and in use;
d) at least 40% glycerol;
e) from 15 to 70 μM dGTP or daGTP;
f) from 15 to 150 μM dATP;
g) from 15 to 150 μM dTTP;
h) from 10 to 18 μM dCTP; and
i) one member selected from the group consisting of:
  i) sufficient ddGTP to provide a ddGTP to dGTP or daGTP ratio of about 5;
  ii) sufficient ddATP to form a ddATP to dATP ratio of about 33;
  iii) ddTTP in a sufficient amount to form a ddTTP to dTTP ratio of about 28; and
  iv) ddCTP in a sufficient amount to form a ddCTP to dCTP ratio of about 22.

2.  The concentrate of claim 1 wherein the thermostable polymerase is <u>Thermus</u> <u>aquaticus</u> polymerase.

3.  The reaction concentrate of claim 2 also comprising:
    a) from 175 to 700μg/mL of concentrate of a protein selected from the group consisting of gelatin and bovine serum albumin;
    b) at least 25 mM of potassium glutamate; and
    c) from 9 to 35 mM of a magnesium salt.

4.  The reaction concentrate of claim 2 also comprising:
    a) from 0.1 to 0.4% amount of a non-ionic surfactant;
    b) from 0.1 to 10 μCi/μL of an alpha-labeled radioactive dNTP;
    c) from 9 to 35 mM of a magnesium salt; and
    d) from 0.1 to 1.0 μM of an oligonucleotide primer having the nucleotide sequence:

    5' CCCAGTCACGACGTTGTAAAACGACGGCCAGTG 3'

5.  A nucleotide sequencing reaction concentrate comprising about:
    a) 0.25 I.U./μL of <u>Thermus</u> <u>aquaticus</u> polymerase;
    b) 350 micro g/mL of bovine serum albumin;
    c) 3.5 mM dithiothreitol;
    d) 17.5 mM magnesium acetate;
    e) 25 mM of potassium glutamate;
    f) 13.1 mM of $K_2HPO_4$;
    g) 4.4 mM of $KH_2PO_4$;
    h) 0.35% of Triton X 100 surfactant;
    i) 40% glycerol; and one mixture selected from the group consisting of:
      A) about 22.7 μM dGTP or daGTP;
      105 μM dATP;
      52.5 μM of dTTP;
      10.5 μM of dCTP; and
      113.7 μM of ddGTP;
      B) about 70 μM of dGTP or daGTP;
      17.5 μM dATP;
      52.5 μM dTTP;
      10.5 μM of dCTP; and
      577 μM of ddATP;
      C) about 70 μM of dGTP or daGTP;
      105 μM of dATP;
      17.5 μM of dTTP;
      10.5 μM of dCTP; and
      490 μM of ddTTP;
      D) about 70 μM of dCTP or daGTP;
      105 μM of dATP;
      52.5 μM of dTTP;

10.5 μM of dCTP; and
231 μM of ddCTP.

6. The reaction concentrate of claim 5 also comprising 0.15 μM of an oligonucleotide primer having the sequence:

5' CCCAGTCACGACGTTGTAAAACGACGGCCAGTG 3'

7. The reaction concentrate of claim 6 also comprising 1 to 10 μCi of alpha-labeled radioactive dCTP per 2 μL of reaction concentrate.

8. The reaction concentrate of claim 7 wherein the radioactive labeled nucleotide is labeled with the element $^{32}P$ or $^{35}S$.

9. A method of oligonucleotide sequencing comprising the steps of :
    a) providing a sequencing vector comprising an unknown nucleotide sequence adjacent to a 3' end of a known nucleotide chain in the vector;
    b) mixing the vector with a primer and a radioactive labeled dNTP; wherein the primer has a nucleotide sequence which is complementary to a portion of the known vector DNA sequence that is adjacent to the unknown DNA sequence;
    c) dividing the mixture b) into four equal volumes in four different vessels labeled G, A, T and C;
    d) providing a series of reaction mixtures by;
        i) adding to the vessel designated G a reaction concentrate according to claim 1 or 2 comprising ddGTP;
        ii) adding to the vessel designated A a reaction concentrate according to claim 1 or 2 comprising ddATP;
        iii) adding to the vessel designated T a reaction concentrate according to claim 1 or 2 comprising ddTTP; and
        iv) adding to the vessel designated C a reaction concentrate according to claim 1 or 2 comprising ddCTP;
    e) incubating the four vessels G, A, T and C at a temperature of 70-74°C from 15 to 30 minutes;
    f) terminating the reactions by the addition of 3 to 5 μL of a reaction stop mixture to each of the four tubes;
    g) separating electrophoretically on a gel the oligonucleotides formed in e) according to length; and
    h) exposing each gel to a photographic film that is sensitive to the radiation generated by the radioactive dNTP to establish the nucleotide sequence of the unknown nucleotide chain.

10. A method according to claim 9 wherein each reaction concentrate G, T, A and C already includes a radioactive dNTP thereby obviating the need to include said radioactive dNTP in step b) of claim 9.

11. The method of claim 9 wherein each reaction concentrate, G, T, A and C, also includes the primer thereby obviating the need to include said primer in step b) of claim 9.

12. The method of claim 9 wherein the sequencing vector has a lacZ nucleotide sequence.

13. The method of claim 9 wherein the sequencing vector is selected from the group consisting of M13mp18 DNA, pUC, pIBI and Bluescript.

14. The method of claim 9 wherein the primer has the nucleotide sequence:

5' CCCAGTCACGACGTTGTAAAACGACGGCCAGTG 3'.

15. The method of claim 9 or 11 in which the sequencing vector is M13mp18 and the reaction concentrates have compositions according to claim 2, 3, 4 or 5.

16. A nucleotide sequencing kit comprising:
    a) a control sequencing vector having a lacZ nucleotide sequence and 1μL of alpha-labeled radioactive dCTP in a first container;
    b) an oligonucleotide primer having the sequence:

5' CCCAGTCACGACGTTGTAAAACGACGGCCAGTG 3'

in a second container; and
c) a reaction concentrate according to claim 2, 3 or 5 in a third container.

17. A nucleotide sequencing kit comprising an oligonucleotide primer having the sequence:

5' CCCAGTCACGACGTTGTAAAACGACGGCCAGTG 3'

in a first container and a reaction concentrate according to claim 2, 3 or 5 in a third container.

18. A nucleotide sequencing kit comprising a reaction concentrate according.to claim 6, 7 or 8.

19. An oligonucleotide having the nucleotide sequence:

5' CCCAGTCCACGACGTTGTAAAACGACGGCCAGTG 3'

20. A method of sequencing double-stranded (dsDNA), comprising the steps of:
a) providing a double-stranded sequencing vector having a lacZ nucleotide sequence into which dsDNA having an unknown nucleotide sequence has been inserted adjacent to a 3' end of a known nucleotide chain in the vector;
b) denaturing the vector with an alkaline solution to form ssDNA;
c) neutralizing the denatured DNA containing solution;
d) mixing the vector with a primer, a magnesium salt and a radioactive labeled dNTP; wherein the primer has a nucleotide sequence which is complementary to at least a portion of known vector DNA sequence that is adjacent to the unknown DNA sequence;
e) dividing the mixture d) into four equal volumes in four different vessels labeled G, A, T and C; and
f) providing a series of reaction mixtures by:
   i) adding to the vessel designated G a reaction concentrate according to claim 1 or 2 comprising ddGTP;
   ii) adding to the vessel designated A a reaction concentrate according to claim 1 or 2 comprising ddATP;
   iii) adding to the vessel designated T a reaction concentrate according to claim 1 or 2 comprising ddTTP;
   iv) adding to the vessel designated C a reaction concentrate according to claim 1 or 2 comprising ddCTP;
g) incubating the four vessels G, A, T and C at a temperature of 70-74°C from 15 to 30 minutes;
h) terminating the reactions by the addition of 3 to 5 µL of a reaction stop mixture to each of the four tubes;
i) separating electrophoretically on a gel the formed oligonucleotides according to length; and
j) exposing each gel to a photographic film which is sensitive to the radiation generated by the radioactive dNTP to establish the sequence of the supercoiled DNA.

**Patentansprüche**

1. Reaktionskonzentrat zur Nukleotid-Sequenzierung mit:
a) einer ausreichenden Menge von thermostabiler Polymerase zur Bereitstellung von 100 bis 500 I.E./mL;
b) 0,3 bis 30 mM eines Reduktionsmittels, ausgewählt aus Dithiothreitol und β-Mercaptoethanol;
c) einer ausreichenden Menge eines Phosphatpuffers zur Aufrechterhaltung eines pH-Wertes von etwa 7,5 sowohl bei der Aufbewahrung als auch bei der Verwendung;
d) mindestens 40 % Glyzerin;
e) 15 bis 70 µM dGTP oder daGTP;
f) 15 bis 150 µM dATP;
g) 15 bis 150 µM dTTP;
h) 10 bis 18 µM dCTP; und
i) einer Komponente, ausgewählt aus der Gruppe bestehend aus;

i) ausreichend ddGTP zur Bereitstellung eines ddGTP zu dGTP-oder daGTP-Verhältnisses von etwa 5;
ii) ausreichend ddATP zur Ausbildung eines ddATP- zu dATP-Verhältnisses von etwa 33;
iii) ddTTP in einer ausreichenden Menge zur Bildung eines ddTTP- zu dTTP-Verhältnisses von etwa 28; und
iv) ddCTP in einer ausreichenden Menge zur Bildung eines ddCTP- zu dCTP-Verhältnisses von etwa 22.

2. Konzentrat nach Anspruch 1, in dem die thermostabile Polymerase <u>Thermus aquaticus</u> Polymerase ist.

3. Reaktionskonzentrat nach Anspruch 2, das weiterhin enthält:
a) 175 bis 700 $\mu$g/mL eines Konzentrates eines Proteins, ausgewählt aus der Gruppe bestehend aus Gelatine und Rinderserumalbumin;
b) mindestens 25 mM Kaliumglutamat; und
c) 9 bis 35 mM eines Magnesiumsalzes.

4. Reaktionskonzentrat nach Anspruch 2, das weiterhin enthält:
a) 0,1 bis 0,4 Mengen-% eines nicht ionogenen oberflächenaktiven Stoffes;
b) 0,1 bis 10 $\mu$Ci/$\mu$L von alpha-markiertem radioaktivem dNTP;
c) 9 bis 35 mM eines Magnesiumsalzes; und
d) 0,1 bis 1,0 $\mu$M eines Oligonukleotid-Starters mit der Nukleotid-Sequenz:

5' CCCAGTCACGACGTTGTAAAACGACGGCCAGTG 3'

5. Reaktionskonzentrat zur Nukleotid- Sequenzierung mit etwa:
a) 0,25 I.E./$\mu$L von <u>Thermus aquaticus</u> Polymerase;
b) 350 micro g/mL von Rinderserumalbumin;
c) 3,5 mM Dithiothreitol;
d) 17,5 mM Magnesiumacetat;
e) 25 mM Kaliumglutamat;
f) 13,1 mM $K_2HPO_4$;
g) 4,4 mM $KH_2PO_4$;
h) 0,35 % Triton X 100 oberflächenaktives Mittel;
i) 40 % Glyzerin; sowie eine Mischung, ausgewählt aus der Gruppe bestehend aus:
A) etwa 22,7 $\mu$M dGTP oder daGTP;
   105 $\mu$M dATP;
   52,5 $\mu$M dTTP;
   10,5 $\mu$M dCTP; und
   113,7 $\mu$M ddGTP;
B) etwa 70 $\mu$M dGTP oder daGTP;
   17,5 $\mu$M dATP;
   52,5 $\mu$M dTTP
   10,5 $\mu$M dCTP; und
   577 $\mu$M ddATP;
C) etwa 70 $\mu$M dGTP oder daGTP;
   105 $\mu$M dATP;
   17,5 $\mu$M dTTP;
   10,5 $\mu$M dCTP; und
   490 $\mu$M ddTTP;
D) etwa 70 $\mu$M dGTP oder daGTP;
   105 $\mu$m dATP;
   52,5 $\mu$m dTTP;
   10,5 $\mu$M dCTP; und
   231 $\mu$M ddCTP.

6. Reaktionskonzentrat nach Anspruch 5, das weiterhin enthält 0,15 $\mu$M eines Oligonukleotid-Starters mit der Sequenz:

5' CCCAGTCACGACGTTGTAAAACGACGGCCAGTG 3'

7. Reaktionskonzentrat nach Anspruch 6, das weiterhin enthält 1 bis 10 µCi von alpha-markiertem radioaktivem dCTP pro 2 µL Reaktionskonzentrat.

8. Reaktionskonzentrat nach Anspruch 7, in dem das radioaktiv markierte Nukleotid mit dem Element $^{32}$P oder $^{35}$S markiert ist.

9. Verfahren zur Oligonukleotid-Sequenzierung mit den Stufen:

a) Bereitstellung eines sequenzierenden Vektors mit einer unbekannten Nukleotid-Sequenz am 3'-Ende einer bekannten Nukleotidkette in dem Vektor;

b) Vermischen des Vektors mit einem Starter und mit einem radioaktiv markierten dNTP; wobei der Starter eine Nukleotid-Sequenz aufweist, die komplementär ist einem Teil der bekannten Vektor-DNA-Sequenz, die benachbart zur unbekannten DNA-Sequenz vorliegt;

c) Aufteilung der Mischung b) in vier gleiche Volumina in vier verschiedenen Gefäßen, markiert G, A, T und C;

d) Bereitstellung einer Reihe von Reaktionsmischungen durch:

i) Zusatz eines Reaktionskonzentrates nach Anspruch 1 oder 2 mit ddGTP zum Gefäß mit der Bezeichnung G;

ii) Zusatz eines Reaktionskonzentrates nach Anspruch 1 oder 2 mit ddATP zum Reaktionsgefäß mit der Bezeichnung A;

iii) Zusatz eines Reaktionskonzentrates nach Anspruch 1 oder 2 mit ddTTP zum Gefäß mit der Bezeichnung T und

iv) Zusatz eines Reaktionskonzentrates nach Anspruch 1 oder 2 mit ddCTP zum Gefäß mit der Bezeichnung C;

e) Inkubierung der vier Gefäße G, A, T und C 15 bis 30 Minuten lang bei einer Temperatur von 70 bis 74°C;

f) Beendigung der Reaktionen durch Zusatz von 3 bis 5 µL einer die Reaktion stoppenden Mischung zu jedem der vier Gefäße;

g) Elektrophoretische Trennung der in e) erzeugten Oligonukleotide auf einem Gel entsprechend der Länge; und

h) Exponierung eines jeden Gels mit einem photographischen Film, der empfindlich ist gegenüber der Strahlung, die durch das radioaktive dNTP erzeugt wird, zur Feststellung der Nukleotid-Sequenz der unbekannten Nukleotidkette.

10. Verfahren nach Anspruch 9, bei dem jedes Reaktionskonzentrat G, T, A und C bereits ein radioaktives dNTP aufweist, wodurch die Notwendigkeit des Einsatzes von radioaktivem dNTP in Stufe b) gemäß Anspruch 9 vermieden wird.

11. Verfahren nach Anspruch 9, bei dem jedes Reaktionskonzentrat G, T, A und C weiterhin den Starter enthält, wodurch der Zusatz des Starters in Stufe b) von Anspruch 9 vermieden wird.

12. Verfahren nach Anspruch 9, bei dem der sequenzierende Vektor eine lacZ-Nukleotid-Sequenz aufweist.

13. Verfahren nach Anspruch 9, bei dem der sequenzierende Vektor ausgewählt ist aus der Gruppe bestehend aus M13mp18 DNA, pUC, pIBI und Bluescript.

14. Verfahren nach Anspruch 9, bei dem der Starter die Nukleotid-Sequenz;

5' CCCAGTCACGACGTTGTAAAACGACGGCCAGTG 3'.

aufweist.

15. Verfahren nach Anspruch 9 oder 11, bei dem der sequenzierende Vektor M13mp18 ist und das Reaktionskonzentrat eine Zusammensetzung gemäß Anspruch 2, 3, 4 oder 5 aufweist.

16. Ein Kitt für die Nukleotid-Sequenzierung mit:

a) einem Vergleichs-Sequenzierungs-Vektor mit einer lacZ-Nukleotid-Sequenz und 1 µL von alpha-markiertem radioaktivem dCTP in einem ersten Behälter;

14

b) einem Oligonukleotid-Starter mit der Sequenz:

5' CCCAGTCACGACGTTGTAAAACGACGGCCAGTG 3'

in einem zweiten Behälter; und
c) einem Reaktionskonzentrat gemäß Anspruch 2, 3 oder 5 in einem dritten Behälter.

17. Kitt für eine Nukleotid-Sequenzierung mit einem Oligonukleotid-Starter mit der Sequenz:

5' CCCAGTCACGACGTTGT AAACGACGGCCAGTG 3'

in einem ersten Behälter und einem Reaktionskonzentrat gemäß Anspruch 2, 3 oder 5 in einem dritten Behälter.

18. Kitt für eine Nukleotid-Sequenzierung mit einem Reaktionskonzentrat nach Anspruch 6, 7 oder 8.

19. Oligonukleotid mit der Nukleotid-Sequenz:

5' CCCAGTCCACGACGTTGTAAAACGACGGCCAGTG 3'

20. Verfahren zur Sequenzierung einer doppel-strangigen (dsDNA), mit den Stufen:
a) Bereitstellung eines doppel-strangigen Sequenzierungs-Vektors mit einer lacZ-Nukleotid-Sequenz, in welche dsDNA mit einer unbekannten Nukleotid-Sequenz benachbart zu einem 3'-Ende einer bekannten Nukleotidkette in dem Vektor eingesetzt worden ist;
b) Denaturierung des Vektors mit einer alkalischen Lösung unter Bildung von ssDNA;
c) Neutralisierung der denaturierten DNA enthaltenden Lösung;
d) Vermischung des Vektors mit einem Starter, einem Magnesiumsalz und einer radioaktiv markierten dNTP; wobei der Starter eine Nukleotid-Sequenz aufweist, die komplementär ist mindestens einem Teil der bekannten Vektor-DNA-Sequenz, die benachbart zu der unbekannten DNA-Sequenz vorliegt;
e) Trennung der Mischung d) in vier gleiche Volumina in vier verschiedenen Gefäßen mit der Bezeichnung G, A, T und C
f) Bereitstellung einer Reihe von Reaktionsmischungen durch:
i) Zusatz eines Reaktionskonzentrates gemäß Anspruch 1 oder 2 mit ddGTP zum Gefäß mit der Bezeichnung G;
ii) Zusatz eines Reaktionskonzentrates gemäß Anspruch 1 oder 2 mit ddATP zum Gefäß mit der Bezeichnung A;
iii) Zusatz eines Reaktionskonzentrates gemäß Anspruch 1 oder 2 mit ddTTP zum Gefäß mit der Bezeichnung T;
iv) Zusatz eines Reaktionskonzentrates gemäß Anspruch 1 oder 2 mit ddCTP zum Gefäß mit der Bezeichnung C;
g) Inkubierung der vier Gefäße G, A, T und C 15 bis 30 Minuten lang bei einer Temperatur von 70 bis 74°C;
h) Beendigung der Reaktionen durch Zusatz von 3 bis 5 µL einer die Reaktion unterbrechenden Mischung zu jedem der vier Gefäße;
i) Elektrophoretische Auftrennung der erzeugten Oligonukleotide nach der Länge auf einem Gel; und
j) Exponierung eines jeden Gels mit einem photographischen Film, der gegenüber der Strahlung empfindlich ist, die durch die radioaktive dNTP erzeugt wird, unter Feststellung der Sequenz der super-gewundenen DNA.

## Revendications

1. Concentrat de réaction pour le séquençage de nucléotides comprenant :
a) une quantité suffisante de polymérase thermostable pour obtenir de 100 à 500 I.U./ml ;
b) de 0,3 à 30 mM d'agent réducteur choisi parmi le dithiothréitol et le β-mercaptoéthanol ;
c) une quantité suffisante de tampon de phosphate pour maintenir un pH d'environ 7,5, à la fois au cours du stockage et lors de l'utilisation ;

d) au moins 40 % de glycérol ;

e) de 15 à 70 μM de dGTP ou de daGTP ;

f) de 15 à 150 μM de dATP ;

g) de 15 à 150 μM de dTTP ;

h) de 10 à 18 μM de dCTP ; et

i) un élément choisi parmi le groupe comprenant :

   i) une quantité suffisante de ddGTP pour obtenir un rapport ddGTP/dGTP ou ddGTP/daGTP d'environ 5 ;

   ii) une quantité suffisante de ddATP pour obtenir un rapport ddATP/dATP d'environ 33 ;

   iii) une quantité suffisante de ddTTP pour obtenir un rapport ddTTP/dTTP d'environ 28 ; et

   iv) une quantité suffisante de ddCTP pour obtenir un rapport ddCTP/dCTP d'environ 22.

2. Concentrat selon la revendication 1, dans lequel la polymérase thermostable est la polymérase <u>Thermus aquaticus</u>.

3. Concentrat selon la revendication 2, comprenant également :

   a) de 175 à 700 μg/ml d'un concentré d'une protéine choisie parmi la gélatine et l'albumine de sérum bovin ;

   b) au moins 25 mM de glutamate de potassium ; et

   c) de 9 à 35 mM d'un sel de magnésium.

4. Concentrat selon la revendication 2, comprenant également :

   a) de 0,1 à 0,4 % d'un agent tensioactif non ionique ;

   b) de 0,1 à 10 μCi/μl de dNTP radioactif marqué en alpha ;

   c) de 9 à 35 mM de sel de magnésium ; et

   d) de 0,1 à 1,0 μM d'un initiateur oligonucléotide ayant la séquence de nucléotides suivante :

   5' CCCAGTCACGACGTTGTAAAACGACGGCCAGTG 3'

5. Concentrat de réaction pour le séquençage des nucléotides comprenant environ :

   a) 0,25 I.U./μl de polymérase <u>Thermus aquaticus</u> ;

   b) 350 μg/ml d'albumine de sérum de bovin ;

   c) 3,5 mM de dithiothréitol ;

   d) 17,5 mM d'acétate de magnésium ;

   e) 25 mM de glutamate de potassium ;

   f) 13,1 mM de $K_2HPO_4$ ;

   g) 4,4 mM de $KH_2PO_4$ ;

   h) 0,35 % d'agent tensioactif Triton X-100 ;

   i) 40 % de glycérol ; et un mélange choisi parmi le groupe comprenant :

   A) environ 22,7 μM de dGTP ou de daGTP ;

      105 μM de dATP ;

      52,5 μM de dTTP ;

      10,5 μM de dCTP ; et

      113,7 μM de ddGTP ;

   B) environ 70 μM de dGTP ou de daGTP ;

      17,5 μM de dATP ;

      52,5 μM de dTTP ;

      10,5 μM de dCTP ; et

      577 μM de ddATP ;

   C) environ 70 μM de dGTP ou de daGTP ;

      105 μM de dATP ;

      17,5 μM de dTTP ;

      10,5 μM de dCTP ; et

      490 μM de ddTTP ;

   D) environ 70 μM de dCTP ou de daGTP ;

      105 μM de dATP ;

      52,5 μM de dTTP ;

10,5 µM de dCTP ; et
231 µM de ddCTP.

6. Concentrat selon la revendication 5 comprenant également 0,15 µM d'un initiateur oligonucléotide ayant la séquence :

5' CCCAGTCACGACGTTGTAAAACGACGGCCAGTG 3'

7. Concentrat selon la revendication 6 comprenant également de 1 à 10 µCi de dCTP radioactif marqué en alpha pour 2 µl de concentrat.

8. Concentrat selon la revendication 7, dans lequel le nucléotide marqué par une substance radioactive est marqué au moyen de l'élément $^{32}$P ou $^{35}$S.

9. Procédé de séquençage des oligonucléotides comprenant les étapes suivantes :
a) obtenir un vecteur de séquençage comprenant une séquence de nucléotides inconnues adjacente à une extrémité 3' d'une chaîne de nucléotides connues dans le vecteur ;
b) mélanger le vecteur avec un initiateur et un dNTP marqué par une substance radioactive ; l'initiateur ayant une séquence de nucléotides qui est complémentaire d'une partie de la séquence connue du vecteur d'ADN qui est adjacente à la séquence d'ADN inconnue ;
c) diviser le mélange b) en quatre volumes égaux dans quatre récipients différents marqués G, A, T et C ;
d) obtenir une série de mélanges de réaction en :
   i) ajoutant au récipient G un concentrat selon la revendication 1 ou 2, comprenant du ddGTP ;
   ii) ajoutant au récipient A un concentrat selon la revendication 1 ou 2, comprenant du ddATP ;
   iii) ajoutant au récipient T un concentrat selon la revendication 1 ou 2, comprenant du ddTTP ; et
   iv) ajoutant au récipient C un concentrat selon la revendication 1 ou 2, comprenant du ddCTP ;
e) faire incuber les quatre récipients G, A, T et C à une température comprise entre 70°C et 74°C pendant une période comprise entre 15 et 30 minutes;
f) ajouter 3 à 5 µl d'un mélange stoppant la réaction dans chacun des quatre tubes ;
g) séparer par électrophorèse sur un gel les oligonucléotides formés dans l'étape e) selon leur longueur ; et
h) exposer chaque gel à un film photographique sensible aux rayonnements générés par le dNTP radioactif afin d'établir la séquence de nucléotides de la chaîne de nucléotides inconnues.

10. Procédé selon la revendication 9, dans lequel chaque concentrat G, T, A et C comprend déjà un dNTP radioactif, ce qui évite d'avoir à incorporer ledit dNTP radioactif dans l'étape b) de la revendication 9.

11. Procédé selon la revendication 9, dans lequel chaque concentrat G, T, A et C comprend également l'initiateur, ce qui évite d'avoir à incorporer ledit initiateur dans l'étape b) de la revendication 9.

12. Procédé selon la revendication 9, dans lequel le vecteur de séquençage comprend une séquence de nucléotides lacZ.

13. Procédé selon la revendication 9, dans lequel le vecteur de séquençage est choisi parmi le groupe comprenant l'ADN M13mp18, pUC, pIBI et Bluescript.

14. Procédé selon la revendication 9, dans lequel l'initiateur comprend la séquence de nucléotides suivante :

5' CCCAGTCACGACGTTGTAAAACGACGGCCAGTG 3'

15. Procédé selon la revendication 9 ou 11, dans lequel le vecteur de séquençage est M13mp18 et les concentrats comprennent les compositions selon les revendications 2, 3, 4 ou 5.

16. Nécessaire pour le séquençage des nucléotides comprenant :
a) un vecteur de séquençage témoin comprenant une séquence de nucléotides lacZ et 1 µl de dCTP radioactif marqué en alpha dans un premier récipient ;
b) un initiateur oligonucléotide ayant la séquence :

5' CCCAGTCACGACGTTGTAAAACGACGGCCAGTG 3'

dans un second récipient ; et

c) un concentrat de réaction selon les revendications 2, 3 ou 5 dans un troisième récipient.

17. Nécessaire pour le séquençage des nucléotides comprenant un initiateur oligonucléotide ayant la séquence :

5' CCCAGTCACGACGTTGTAAAACGACGGCCAGTG 3'

dans un premier récipient et un concentrat selon les revendications 2, 3 ou 5 dans un troisième récipient.

18. Nécessaire pour le séquençage des nucléotides comprenant un concentrat selon les revendications 6, 7 ou 8.

19. Oligonucléotide comprenant la séquence de nucléotides suivante :

5' CCCAGTCACGACGTTGTAAAACGACGGCCAGTG 3'

20. Procédé de séquençage du dsADN double brins, comprenant les étapes suivantes :
   a) obtenir un vecteur de séquençage à double brins comprenant une séquence de nucléotides lacZ dans lequel le dsADN comprenant une séquence de nucléotides inconnues a été inséré de façon adjacente à une extrémité 3' d'une chaîne de nucléotides connues dans le vecteur ;
   b) dénaturer le vecteur en utilisant une solution basique pour former le ssADN ;
   c) neutraliser la solution contenant l'ADN dénaturé ;
   d) mélanger le vecteur avec un initiateur, un sel de magnésium et un dNTP marqué par une substance radioactive ; dans lequel l'initiateur comprend une séquence de nucléotides qui est complémentaire d'au moins une partie de la séquence connue du vecteur d'ADN qui est adjacente à la séquence d'ADN inconnue ;
   e) diviser le mélange d) en quatre volumes égaux dans quatre récipients différents marqués G, A, T et C ; et
   f) obtenir une série de mélanges de réaction en :
      i) ajoutant au récipient G un concentrat selon la revendication 1 ou 2, comprenant du ddGTP ;
      ii) ajoutant au récipient A un concentrat selon la revendication 1 ou 2, comprenant du ddATP ;
      iii) ajoutant au récipient T un concentrat selon la revendication 1 ou 2, comprenant du ddTTP ; et
      iv) ajoutant au récipient C un concentrat selon la revendication 1 ou 2, comprenant du ddCTP ;
   g) faire incuber les quatres récipients G, A, T et C à une température comprise entre 70°C et 74°C pendant une période comprise entre 15 et 30 minutes;
   h) ajouter 3 à 5 µl d'un mélange stoppant la réaction dans chacun des quatre tubes ;
   i) séparer par électrophorèse sur gel les oligonucléotides formés selon leur longueur ; et
   j) exposer chaque gel à un film photographique sensible aux rayonnements générés par le dNTP radioactif pour établir la séquence de l'ADN en superhélice.